# EUROPÄISCHE PATENTSCHRIFT

(11) **EP 1 253 146 B1**
(45) Veröffentlichungstag und Bekanntmachung des Hinweises auf die Patenterteilung: **10.09.2003**
(21) Anmeldenummer: 02007597.4
(22) Anmeldetag: 04.04.2002
(51) Int. Cl.: C07D 307/34

(54) **Verfahren zur Herstellung von substituierten Furancarboxylaten**
Process for the synthesis of substituted furancarboxylates
Procédé pour la préparation des furancarboxylates substituées

(30) Priorität: 27.04.2001 AT 6822001
(43) Veröffentlichungstag der Anmeldung: 30.10.2002
(73) Patentinhaber: DSM Fine Chemicals Austria Nfg GmbH & Co KG, 4021 Linz (AT)
(72) Erfinder: Pöchlauer, Peter, 4040 Linz (AT); Ganglberger, Thorsten, 4020 Linz (AT); Mayrhofer, Herbert, 4210 Engerwitzdorf (AT)
(74) Vertreter: Lindinger, Ingrid

(56) Entgegenhaltungen:
- VERONESE A C ET AL: "TIN (IV) CHLORIDE-PROMOTED REACTIONS OF ALPHA-HYDROXY NITRILES WITH BETA-DICARBONYL COMPOUNDS SYNTHESIS OF 4-AMINO-2,5-DIHYDRO-2-FURANONES AND THEIR CONVERSION INTO TETRONIC ACID DERIVATIVES" HETEROCYCLES, XX, XX, Bd. 11, Nr. 32, 1991, Seiten 2205-2215, XP008003483 ISSN: 0385-5414
- TRAHANOVSKY W S ET AL: "Effects of a-tert-butyl group substitution on the reactivity and dimerization products of furan-based o-quinodimethanes" JOURNAL OF ORGANIC CHEMISTRY, Bd. 59, 1994, Seiten 2594-2598, XP002202534 USA
- EL-SHAFEL A K ET AL: "synthesis of thieno(2,3-b)thiophenes and related structures" PHOSPHORUS, SULFUR, AND SILICON, Bd. 73, 1992, Seiten 15-25, XP002202535 USA

## Beschreibung

Die Erfindung betrifft die Herstellung von substituierten Furancarboxylaten aus Cyanhydrinen.

Furancarboxylate finden in vielen Bereichen Anwendung und werden beispielsweise als Agro- oder Pharmawirkstoffe, wie etwa kardiovaskuläre Mittel oder Antihypertensiva, eingesetzt.

Cyanhydrine, die als Ausgangsprodukt zur Herstellung der substituierten Furancarboxylate dienen, werden etwa zur Synthese von alpha-Hydroxysäuren, alpha-Hydroxyketonen, beta-Aminoalkoholen, die zur Gewinnung biologisch wirksamer Stoffe, z. B. pharmazeutischer Wirkstoffe, Vitamine oder auch pyrethroider Verbindungen Verwendung finden, eingesetzt.
Aus Heterocycles, Vol. 32, No. 11, 1991, S. 2205-2215 ist weiters ein Verfahren bekannt, bei welchem racemische α-Hydroxynitrile durch intermolekulare Reaktion mit β-Dicarbonylverbindungen, wie etwa Methyl- oder Ethylacetoacetat, in Gegenwart von stöchiometrischen Mengen an Zinn(IV)chlorid direkt zu den entsprechenden Furanonen, etwa zu den korrespondierenden 3-Acyl-4-amino-2(5H)-furanonen, in 50-60%iger Ausbeute umgesetzt werden.

Unerwarteterweise wurden bei der Umsetzung von α-Hydroxynitrilen nicht die korrespondierenden Acylfuranone erhalten, sondern Furancarboxylate.

Gegenstand der Erfindung ist demnach ein Verfahren zur Herstellung von substituierten Furancarboxylaten der Formel (I) 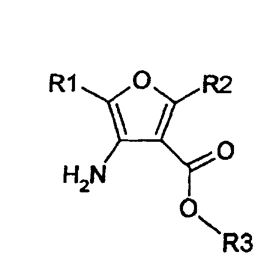 in der R1 einen gegebenenfalls durch unter den Reaktionsbedingungen inerte Gruppen substituierten, linearen, verzweigten oder cyclischen C₁-C₁₈-Alkyl-, C₂-C₁₈ Alkenyl- oder C₂-C₁₈ Alkinylrest, einen gegebenenfalls substituierten aromatischen oder heteroaromatischen Rest, und R2 und R3 unabhängig voneinander einen C1-C4-Alkylrest bedeuten, das dadurch gekennzeichnet ist, dass α-Hydroxynitrile der Formel (II) 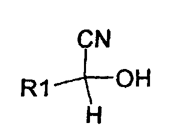 in der R1 wie oben definiert ist, mit β-Dicarbonylverbindungen der Formel (III) 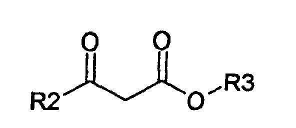 in der R2 und R3 wie oben definiert sind, in Gegenwart von Metallhalogeniden als Katalysatoren bei einer Temperatur von 0°C bis 120°C in einem unter den Reaktionsbedingungen inerten, organischem Lösungsmittel umgesetzt werden, nach erfolgter Reaktion der Katalysator abgetrennt und das entsprechende Furancarboxylat isoliert wird.

Das erfindungsgemäße Verfahren betrifft die Herstellung von substituierten Furancarboxylaten der Formel (I).

Dabei wird erfindungsgemäß von α-Hydroxynitrilen der Formel (II) ausgegangen, die in Form ihrer Racemate oder als enantiomerenreine oder -angereicherte (S)- oder (R)-Hydroxynitrile eingesetzt werden können.
Die eingesetzten Hydroxynitrile werden durch Addition von Blausäure, welche als solche dem Reaktionsgemisch zugesetzt oder in situ aus einem Cyanidgruppendonor erzeugt wird, an die entsprechenden Carbonylverbindungen hergestellt.
Die basen- oder enzymkatalysierte Addition von Blausäure an die entsprechenden Carbonylverbindungen kann dabei analog dem Stand der Technik, beispielsweise analog EP 0 951 561, EP 0 927 766, EP 0 632 130, EP 0547 655, EP 0 326 063 u.s.w., erfolgen.
Beispiele für Carbonylverbindungen, die gemäß dem Stand der Technik eingesetzt werden sind aliphatische, aromatische oder heteroaromatische Carbonylverbindungen, bei denen das Carbonylkohlenstoffatom ungleich substituiert ist. Unter aliphatischen Carbonylverbindungen sind gesättigte oder ungesättigte, geradkettige, verzweigte oder cyclische Carbonylverbindungen zu verstehen. Die Carbonylverbindungen können gesättigt oder ein- oder mehrfach ungesättigt sein. Sie können unsubstituiert, oder ein- oder mehrfach durch unter den Reaktionsbedingungen inerte Gruppen, beispielsweise durch gegebenenfalls substituierte Aryl- oder Heteroarylgruppen, wie Phenyl- oder Indolylgruppen, durch Halogen-, Ether-, Alkohol-, Acyl-, Carbonsäure-, Carbonsäureester-, Nitro- oder Azidogruppen substituiert sein.
Beispiele für aromatische oder heteroaromatische Carbonylverbindungen sind Benzaldehyd, bzw. verschieden substituierte Benzaldehyde wie etwa 3,4-Difluorbenzaldehyd, 3-Phenoxybenzaldehyd, 4-Fluor-3-phenoxybenzaldehyd, Hydroxybenzaldehyd, Methoxybenzaldehyd, weiters Furfural, Methylfurfural, Anthracen-9-carbaldehyd, Furan-3-carbaldehyd, Indol-3-carbaldehyd, Naphthalin-1-carbaldehyd, Phthaldialdehyd, Pyrazol-3-carbaldehyd, Pyrrol-2-carbaldehyd, Thiophen-2-carbaldehyd, Isophthalaldehyd oder Pyridinaldehyde, Thienylaldehyde u.s.w..

Die als Ausgangsprodukt dienenden Cyanhydrine bzw. Hydroxynitrile weisen die Formel (II) auf, in der R1 einen gegebenenfalls durch unter den Reaktionsbedingungen inerte Gruppen substituierten, linearen, verzweigten oder cyclischen C₁-C₁₈-Alkyl-, Alkenyl- oder Alkinylrest, einen gegebenenfalls substituierten aromatischen oder heteroaromatischen Rest bedeutet.
R1 kann somit ein linearer, verzweigter oder cyclischer C₁-C₁₈-Alkyl-, C₂-C₁₈ Alkenyl- oder C₂-C₁₈ Alkinylrest, bevorzugt ein C₂-C₁₂-Alkyl-, Alkenyl- oder Alkinylrest und besonders bevorzugt ein C₂-C₈-Alkyl-, Alkenyl- oder Alkinylrest sein, wie etwa Ethyl, Ethenyl, n-Propyl, Propenyl, Propinyl, i-Propyl, Cyclopropyl, n-Butyl, t.-Butyl, 1-Butenyl, 2-Butenyl, 3-Butenyl, 1-Methylpropyl, 2-Methylpropyl, 1,1-Dimethylethyl, 2-Methylpropenyl, Butinyl, n-Pentyl, 1-Pentenyl, 2-Pentenyl, 3-Pentenyl, 4-Pentenyl, Cyclopentyl,1-Methylbutyl, 2-Methylbutyl, 3-Methylbutyl, 2,2-Dimetylpropyl, 1-Ethylpropyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, Hexyl, 1-Hexenyl, 2-Hexenyl, 3-Hexenyl, 4-Hexenyl, 5-Hexenyl, Cydohexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 4-Methylbutyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-2-butenyl, 2,2-Dimethyl-3-butenyl, 1-Ethyl-1-butenyl, 2-Ethyl-3-butenyl, 1,1,2-Trimethyl-2-propenyl, 1-Ethyl-2-methyl-2-propenyl, n-Heptyl, 1-Heptenyl, 2-Heptenyl, 3-Heptenyl, 4-Heptenyl, 5-Heptenyl, 6-Heptenyl, Cycloheptyl, n-Octyl, 1-Octenyl, 2-Octenyl, 3-Octenyl, 4-Octenyl, 5-Octenyl, 6-Octenyl, 7-Octenyl, n-Nonyl, Nonenyl, n-Decyl, Decenyl, n-Dodecyl u.s.w.
R1 kann weiters ein Aryl- oder ein Heteroarylrest sein. Unter Arylrest sind Reste mit 6-20 C-Atomen im Ring oder im Ringsystem zu verstehen. Bevorzugte Arylreste sind Phenyl oder Naphthyl. Unter Heteroaryl sind einfache oder kondensierte aromatische Ringsysteme mit einem oder mehreren heteroaromatischen 3-7gliedrigen Ringen, die ein oder mehrere Heteroatome der Gruppe N, S oder O enthalten, zu verstehen. Bevorzugt sind dabei Furan, Pyridin, Imidazol, Pyrimidin, Purin, Pyrazin oder Chinolin.
R1 kann dabei gegebenenfalls durch eine oder mehrere unter den Reaktionsbedingungen inerten Gruppen substituiert sein. Geeignete Substituenten sind beispielsweise Halogen, wie Fluor, Chlor oder Brom, Thio, Nitro, gegebenenfalls substituierte Aminogruppen, Hydroxy, C₁-C₆-Alkyl, C₁-C₆-Alkoxy, C₂-C₆-Alkenyl, C₂-C₆-Alkenyloxy, C₂-C₆-Alkinyl, gegebenenfalls substituiertes Phenyl, u.s.w.

Bevorzugt bedeutet R1 einen gegebenenfalls durch Fluor, Chlor oder Brom, Hydroxy oder gegebenenfalls substituiertes Phenyl substituierten, linearen oder verzweigten C₂-C₁₈-Alkylrest oder gegebenenfalls durch Fluor, Chlor oder Brom, Hydroxy, C₁-C₆-Alkyl, C₁-C₆-Alkoxy substituierten Phenyl- oder Naphtylrest.

Besonders bevorzugt bedeutet R1 einen gegebenenfalls durch Chlor, Hydroxy, C₁-C₄-Alkyl oder C₁-C₄-Alkoxy substituierten Phenylrest.

Das Hydroxynitril der Formel (II) wird erfindungsgemäß mit einer β-Dicarbonylverbindungen der Formel (III) umgesetzt.

In der Formel (III) bedeuten R2 und R3 unabhängig voneinander einen C₁-C₄-Alkylrest. Unter Alkylrest ist dabei ein linearer oder verzweigter Alkylrest mit 1-4 C-Atomen, wie Methyl, Ethyl, n-Propyl, i-Propyl, n-Butyl oder t.-Butyl, zu verstehen. Bevorzugt bedeuten R2 und R3 Methyl oder Ethyl, besonders bevorzugt bedeutet R2 Methyl und R3 Ethyl.
Das molare Verhältnis von Hydroxynitril der Formel (II) zu β-Dicarbonylverbindung der Formel (III) beträgt 1:1 bis 1:3, bevorzugt 1:1 bis 1:2 und besonders bevorzugt 1:1 bis 1:1,5.

Die Umsetzung erfolgt in Anwesenheit von Metallhalogeniden als Katalysatoren. Geeignete Metallhalogenide sind beispielsweise Sn-, Ti- oder Zr-halogenide, wie etwa SnCl₄, SnBr₄, SnJ₄, TiCl₄, TiBr₄, ZrCl₄ u.s.w.. Bevorzugt werden Sn-, Ti- oder Zr-chloride, besonders bevorzugt SnCl₄ eingesetzt. Der Katalysator wird dabei bevorzugt in einer stöchiometrischen Menge, bezogen auf das Nitril, eingesetzt. Gegebenenfalls kann jedoch auch ein bis zu 20%iger molarer Überschuss an Katalysator verwendet werden.

Als Lösungsmittel eignen sich solche, die unter den Reaktionsbedingungen inert sind. Beispiele dafür sind halogenierte oder halogenfreie Kohlenwasserstoffe, wie etwa Dichlormethan oder Toluol; sowie Ethylacetat.

Die Reaktionstemperaturen liegen zwischen 0°C und 120°C, bevorzugt zwischen 10 und 115°C und besonders bevorzugt zwischen 15 und 110°C.

Die Umsetzung der Verbindungen der Formel (II) mit einer Verbindung der Formel (III) erfolgt dabei bevorzugt unter Inertgasatmosphäre, beispielsweise unter Stickstoff- oder Argonatmosphäre.

Das Reaktionsgemisch wird während der Umsetzung bei der gewünschten Reaktionstemperatur gerührt. Die Reaktionsdauer liegt je nach Wahl der Edukte und der Reaktionsbedingungen zwischen etwa 1 und 50 Stunden.

Zur Aufarbeitung des Reaktionsgemisches wird ein Carbonat oder ein Hydrogencarbonat, wie etwa Natriumcarbonat, Natriumhydrogencarbonat, Ammonimcarbonat, Kaliumcarbonat oder Kaliumhydrogencarbonat, zugegeben, wobei sich ein Niederschlag an Metallhydroxid, je nach eingesetztem Katalysator, bildet und eine Trennung des Reaktionsgemisches in 2 Phasen erfolgt. Das Zweiphasengemisch wird sodann zur Entfernung des Niederschlages filtriert und die wässrige Phase beispielsweise 1 bis 6mal extrahiert. Als Extraktionsmittel eignen sich mit Wasser nicht mischbare Lösungsmittel, wie etwa Ethylacetat oder Dichlormethan.
Die vereinigten organischen Phasen werden gegebenenfalls getrocknet, beispielsweise mit Natriumsulfat oder Magnesiumsulfat, und das Lösungsmittel im Vakuum abdestilliert.
Der erhaltene Rückstand wird sodann in einem geeigneten mit Wasser mischbaren Lösungsmittel, wie etwa in einem C₁-C₃-Alkohol, beispielsweise Methanol, oder in Tetrahydrofuran, gelöst und durch Zugabe von Wasser auskristallisiert, worauf das gewünschte Furancarboxylat der Formel (I) als Feststoff, der sodann getrocknet wird, erhalten wird.

Durch das erfindungsgemäße Verfahren werden die gewünschten Furancarboxylate in Ausbeuten von bis zu 90% der Theorie erhalten.

### Beispiel 1

Einer Lösung aus 2,7 g (20 mmol) Mandelsäurenitril und 3,12 g (24 mmol) Acetessigsäureethylester in 16 ml Ethylacetat wurde unter Argonatmosphäre 2,3 ml (20mmol) SnCl₄ zugefügt. Dabei stieg die Temperatur des Reaktionsgemisches auf ca. 70°C und das Gemisch färbte sich rot.
Anschließend wurde 44 Stunden bei Raumtemperatur gerührt. Dann wurden 55 ml Natriumcarbonatlösung zugesetzt und eine weitere Stunde gerührt, wobei ein weißer Niederschlag von Zinnhydroxid ausfiel und sich 2 Phasen bildeten. Das Zweiphasengemisch wurde über Celite filtriert und die wässrige Phase 4-mal mit Ethylacetat extrahiert.
Die vereinten organischen Phasen wurden mit Natriumsulfat getrocknet und das Lösungsmittel im Vakuum abdestilliert. Der orange Rückstand wurde in Methanol aufgelöst und durch Zugabe von Wasser auskristallisiert. Der nunmehr gelbe kristalline Feststoff, das Ethyl-4-Amino-2-methyl-5-phenyl-furan-3-carboxylat, wurde abschließend getrocknet.
- Ausbeute an Ethyl-4-Amino-2-methyl-5-phenyl-furan-3-carboxylat:: 4,35g (88,5% d.Th.)
- 13C-NMR (DMSO) δ:: 14.47, 14.96, 60.29, 108.01, 122.19, 124.95, 128.99, 131.06, 131.40, 132.33, 157.35, 157.35, 164.53
- ¹H-NMR (DMSO) δ:: 1.33 (t, 3H, CH₃), 2.55 (s, 3H, CH₃), 4.31 (q, 2H, CH₂), 4.78 (br, 2H, NH₂), 7.12-7.54 (m, 5H, Phe)
- IR-Spektrum (KBr) νₘₐₓ:: 3433, 3351, 3048, 2977, 1685, 1626, 1600, 1498, 1460, 1440, 1374, 1343, 1284, 1125, 1018

## Patentansprüche

1. Verfahren zur Herstellung von substituierten Furancarboxylaten der Formel (I) 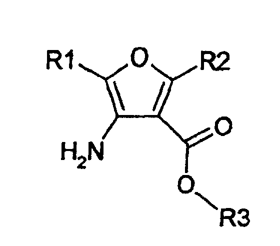 in der R1 einen gegebenenfalls durch unter den Reaktionsbedingungen inerte Gruppen substituierten, linearen, verzweigten oder cyclischen C₁-C₁₈-Alkyl-, C₂-C₁₈ Alkenyl- oder C₂-C₁₈ Alkinylrest, einen gegebenenfalls substituierten aromatischen oder heteroaromatischen Rest, und R2 und R3 unabhängig voneinander einen C₁-C₄-Alkylrest bedeuten, **dadurch gekennzeichnet, dass** α-Hydroxynitrile der Formel (II) 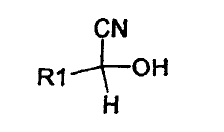 in der R1 wie oben definiert ist, mit β-Dicarbonylverbindungen der Formel (III) 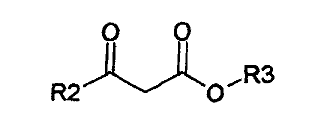 in der R2 und R3 wie oben definiert sind, in Gegenwart von Metallhalogeniden als Katalysatoren bei einer Temperatur von 0°C bis 120°C in einem unter den Reaktionsbedingungen inerten, organischem Lösungsmittel umgesetzt werden und nach erfolgter Reaktion der Katalysator abgetrennt und das entsprechende Furancarboxylat isoliert wird.

2. Verfahren nach Anspruch 1, **dadurch gekennzeichnet, dass** die Hydroxynitrile der Formel II in Form von Racematen oder als enantiomerenreine oder -angereicherte (S)- oder (R)-Hydroxynitrile eingesetzt werden.

3. Verfahren nach Anspruch 1, **dadurch gekennzeichnet, dass** R1 einen gegebenenfalls durch Fluor, Chlor oder Brom, Hydroxy oder gegebenenfalls substituiertes Phenyl substituierten, linearen oder verzweigten C₁-C₁₈-Alkylrest oder einen gegebenenfalls durch Fluor, Chlor oder Brom, Hydroxy, C₁-C₆-Alkyl, C₁-C₆-Alkoxy substituierten Phenyl- oder Naphtylrest bedeutet.

4. Verfahren nach Anspruch 1, **dadurch gekennzeichnet, dass** als Katalysatoren Sn-, Ti- oder Zr-halogenide eingesetzt werden.

5. Verfahren nach Anspruch 4, **dadurch gekennzeichnet, dass** als Katalysatoren Sn-, Ti- oder Zr-chloride eingesetzt werden.

6. Verfahren nach Anspruch 1, **dadurch gekennzeichnet, dass** als Lösungsmittel ein halogenierter oder halogenfreier Kohlenwasserstoff oder Ethylacetat verwendet wird.

7. Verfahren nach Anspruch 1, **dadurch gekennzeichnet, dass** zur Abtrennung des Katalysators ein Carbonat oder ein Hydrogencarbonat zugesetzt wird, worauf das entsprechende Metallhydroxid ausfällt und eine Trennung des Reaktionsgemisches in 2 Phasen erfolgt.

8. Verfahren nach Anspruch 7, **dadurch gekennzeichnet, dass** das erhaltene Zweiphasengemisch zur Entfernung des Niederschlages abfiltriert wird und die wässrige Phase mit einem mit Wasser nicht mischbaren Extraktionsmittel extrahiert wird.

9. Verfahren nach Anspruch 7-8, **dadurch gekennzeichnet, dass** die vereinten organischen Phasen gegebenenfalls getrocknet werden, das Lösungsmittel abdestilliert und der Rückstand in einem mit Wasser mischbaren Lösungsmittel gelöst wird, worauf das entsprechende Furancarboxylat durch Wasserzugabe auskristallisiert wird.

## Claims

1. Process for preparing substituted furancarboxylates of the formula (I) 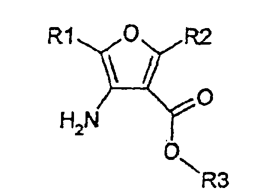 where R1 is a linear, branched or cyclic C₁-C₁₈-alkyl, C₂-C₁₈-alkenyl or C₂-C₁₈-alkynyl radical, each of which is optionally substituted by groups which are inert under the reaction conditions, an optionally substituted aromatic or heteroaromatic radical, and R2 and R3 are each independently a C₁-C₄-alkyl radical, **characterized in that** α-hydroxy nitriles of the formula (II) 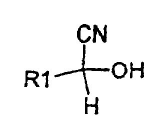 where R1 is as defined above are reacted with β-dicarbonyl compounds of the formula (III) 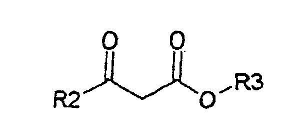 where R2 and R3 are each as defined above, in the presence of metal halides as catalysts at a temperature of 0°C to 120°C in an organic solvent which is inert under the reaction conditions, and, after completed reaction, the catalyst is removed and the corresponding furancarboxylate is isolated.

2. Process according to Claim 1, **characterized in that** the hydroxy nitriles of the formula II are used in the form of racemates or as enantiomerically pure or enantiomerically enriched (S)- or (R)-hydroxy nitriles.

3. Process according to Claim 1, **characterized in that** R1 is an optionally fluorine-, chlorine- or bromine-, hydroxy- or optionally substituted phenyl-substituted, linear or branched C₁-C₁₈-alkyl, or is an optionally fluorine-, chlorine- or bromine-, hydroxy-, C₁-C₆-alkyl-, C₁-C₆-alkoxy-substituted phenyl or naphthyl radical.

4. Process according to Claim 1, **characterized in that** the catalysts used are tin halides, titanium halides or zirconium halides.

5. Process according to Claim 4, **characterized in that** the catalysts used are tin chlorides, titanium chlorides or zirconium chlorides.

6. Process according to Claim 1, **characterized in that** the solvent used is a halogenated or halogen-free hydrocarbon or ethyl acetate.

7. Process according to Claim 1, **characterized in that** the catalyst is removed by adding a carbonate or a hydrogen carbonate, whereupon the corresponding metal hydroxide precipitates out and the reaction mixture separates into 2 phases.

8. Process according to Claim 7, **characterized in that** the precipitate is removed by filtering the biphasic mixture obtained and the aqueous phase is extracted using a water-immiscible extractant.

9. Process according to Claim 7-8, **characterized in that** the combined organic phases are optionally dried, the solvent is distilled off and the residue is dissolved in a water-miscible solvent, whereupon the corresponding furancarboxylate crystallizes out by the addition of water.

## Revendications

1. Procédé de préparation de furannecarboxylates substitués de la formule I : 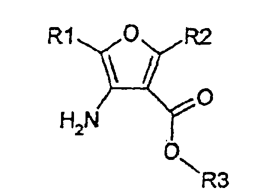 dans laquelle R1 représente un groupe alkyle en C₁-C₁₈, alcényle en C₂-C₁₈ ou alcynyle en C₂-C₁₈, linéaire, ramifié ou cyclique, le cas échéant substitué par des radicaux inertes dans les conditions de réaction, un groupe aromatique ou hétéroaromatique le cas échéant substitué, et R2 et R3 représentent indépendamment l'un de l'autre, un groupe alkyle en C₁-C₄, qui est **caractérisé en ce que** l'on fait réagir l'α-hydroxy-nitrile de la formule (II) : 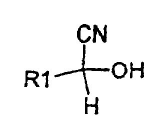 dans laquelle R1 est tel que défini ci-dessus, avec des composés β-dicarbonylés de la formule (III) : 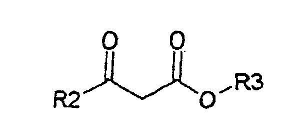 dans laquelle R2 et R3 sont tels que définis ci-dessus, en présence d'halogénures métalliques comme catalyseur, à une température allant de 0°C à 120°C dans un solvant organique, inerte dans les conditions de réaction, après la réaction, on sépare le catalyseur et on isole le furannecarboxylate correspondant.

2. Procédé selon la revendication 1, **caractérisé en ce que** les hydroxynitriles de la formule II sont mis en oeuvre sous la forme de racémates ou de (S)- ou (R)-hydroxynitrile énantiomériquement pur ou énantiomériquement enrichi.

3. Procédé selon la revendication 1, **caractérisé en ce que** R1 représente un groupe alkyle en C₁-C₁₈ linéaire ou ramifié, le cas échéant substitué par fluor, chlore ou brome, hydroxyle ou phényle le cas échéant substitué, ou représente un reste phényle ou naphtyle, le cas échéant substitué par fluor, chlore ou brome, hydroxyle, alkyle en C₁-C₆, alcoxy en C₁-C₆.

4. Procédé selon la revendication 1, **caractérisé en ce que** l'on met en oeuvre comme catalyseur, des halogénures de Sn, Ti ou Zr.

5. Procédé selon la revendication 4, **caractérisé en ce que** l'on met en oeuvre comme catalyseur, des chlorures de Sn, Ti ou Zr.

6. Procédé selon la revendication 1, **caractérisé en ce que** l'on utilise comme solvant, un hydrocarbure halogéné ou non halogéné ou l'acétate d'éthyle.

7. Procédé selon la revendication 1, **caractérisé en ce que** pour la séparation du catalyseur, on ajoute un carbonate ou un hydrogénocarbonate, où l'hydroxyde métallique correspondant précipite et il se produit une séparation du mélange réactionnel en deux phases.

8. Procédé selon la revendication 7, **caractérisé en ce que** le mélange biphasique obtenu est filtré pour l'élimination du résidu et on extrait la phase aqueuse avec un agent d'extraction non miscible à l'eau.

9. Procédé selon les revendications 7-8, **caractérisé en ce que** les phases organiques rassemblées sont le cas échéant séchées, le solvant est distillé et le résidu est dissous dans un solvant miscible à l'eau, où le furannecarboxylate approprié cristallise par addition d'eau.
